Europäisches Patentamt

European Patent Office

Office européen des brevets

(19)

(11) Publication number : **0 593 176 A2**

(12) **EUROPEAN PATENT APPLICATION**

(21) Application number : **93307656.4**

(22) Date of filing : **28.09.93**

(51) Int. Cl.⁵ : **A61K 39/08**

(30) Priority : **28.09.92 US 951604**

(43) Date of publication of application :
**20.04.94 Bulletin 94/16**

(84) Designated Contracting States :
**AT BE CH DE DK ES FR GB GR IE IT LI LU MC NL PT SE**

(71) Applicant : **WISCONSIN ALUMNI RESEARCH FOUNDATION**
**P.O. Box 7365**
**Madison, WI 53705-7365 (US)**

(72) Inventor : **Schantz, Edward J.**
**5102 South Hill Drive**
**Madison WI 53705 (US)**
Inventor : **Goodnough, Michael C.**
**6914 Harvest Hill Road**
**Madison WI 53717 (US)**
Inventor : **Johnson, Eric A.**
**3901 Council Court**
**Madison WI 53711 (US)**

(74) Representative : **Ellis-Jones, Patrick George Armine**
**J.A. KEMP & CO. 14 South Square Gray's Inn**
**London WC1R 5LX (GB)**

(54) **Pharmaceutical compositions containing botulinum toxin and method of preparation.**

(57)    A pharmaceutical composition contains active lyophilized botulinum toxin type A, no sodium chloride and less than about 25 % inactive toxin is disclosed along with a method of preparing it.

EP 0 593 176 A2

EP 0 593 176 A2

## Field of the Invention

The present invention relates to botulinum toxin. More particularly, it relates to novel pharmaceutical compositions containing botulinum toxin and a method of preparing the compositions.

## Background of the Invention

The most serious form of bacterial food poisoning is botulism which is caused by neurotoxins produced by *Clostridium botulinum*. The toxins are absorbed from the intestinal tract and transported via the circulatory system to motor nerve synapses where their action blocks normal neural transmissions. Various serotypes of *C. botulinum* produce neurotoxins with similar toxic activity but which differ antigenetically. Type A is the predominant toxin in cases of botulism in the United states, and type B toxin is most prevalent in Europe.

Crystalline botulinum toxin type A prepared in 1979 by E. J. Schantz of the Food Research Institute/Department of Food Microbiology and Toxicology, University of Wisconsin, Madison, Wisconsin, has been used medicinally in the treatment of hyperactive muscle movement disorders, such as strabismus and other involuntary muscle disorders. Treatment involves injection of nanogram quantities (1 ng is equal to 30 mouse 50% lethal doses [30 U]) of the toxin directly into the hyperactive muscles. The toxin inhibits the release of acetylcholine from the motor neuron across the synaptic junction, causing a decrease in the hyperactivity of the injected muscles.

Botulinum toxin has become one of the most effective treatments for various types of dystonias, or involuntary muscle contractions, for many thousands of people. During the last few years, a limited number of dystonia patients have developed antibodies to the toxin resulting in ineffective treatment with the toxin, particularly for patients treated for spasmodic torticollis which requires larger and sometimes more frequent doses of the toxin by injection.

Currently, the commercial preparation of the crystalline type A botulinum is an ultrafiltered preparation containing the toxin lyophilized with physiological saline and human serum albumin (HSA) in small ampoules and kept under vacuum. Each vial of the commercial toxin product is prepared to contain 100 U of toxin. The method of preparing the commercially available pharmaceutical composition results in 80 to 90 percent loss of the activity of the toxin that causes the paralyzing action. As a result, to be able to dispense 100 U of the active toxin, it is necessary to start with 500 to 1000 U because of the 80 to 90% loss which occurs during preparation. The remaining 10 or 20 percent of activity is sufficient for treatment, but inactivated toxin is injected along with the active toxin. It is believed that the inactive toxin which is present acts as a toxoid and the result is both the active and inactive toxin can contribute to the undesirable formation of antibodies and the neutralization of the toxin injected into the body. The fact that antibodies to the toxin have been formed in a limited number of patients has been verified by the U.S. Center for Disease Control and other medical units.

Although the commercially available preparation is very useful, it would be beneficial to have an improved composition and method of preparation of botulinum toxin which is substantially free of inactive toxin.

## Brief Summary of the Invention

It is an object of the present invention to disclose novel pharmaceutical composition that contains active botulinum toxin and which are substantially free of inactive botulinum toxin.

It is a further object to disclose a novel method of preparing such pharmaceutical composition.

The novel pharmaceutical compositions of the present invention contain about 100 U of active crystalline botulinum toxin type A and an effective amount of a stabilizer, such as serum albumin. The compositions are sodium chloride-free and substantially free of inactive toxin.

The novel method of preparing the pharmaceutical compositions of the present invention comprises dissolving *C. botulinum* type A toxin in distilled water or a suitable buffer (pH about 5.0 to about 6.8) containing a stabilizing amount of a protein, such as serum albumin, filtering the solution, placing the resulting solution in a container and freezing the solution in liquid nitrogen. The frozen solution is then dried by lyophilization to obtain the desired pharmaceutical composition.

At the time of use, the lyophilized compositions can be reconstituted by aseptically introducing physiological saline into the container to obtain a water clear solution. The desired amount of the solution (e.g. 0.1 ml) is then injected using a suitable needle, such as an electromyographic needle, into the proper or hyperactive muscle.

2

## Description of the Preferred Embodiment

The preferred embodiment of the compositions of the present invention is a lyophilized product containing about 100 U of active botulinum toxin type A or a number of units appropriate for medical treatment and about 0.5% of human serum albumin in a sealed ampoule under vacuum. It contains no sodium chloride and is substantially free (i.e. contains less than 25%) of inactive toxin.

The preferred method of preparing the compositions of the present invention comprises dissolving the botulinum type A toxin with a solubilizing amount of sodium phosphate buffer (pH 6.2-6·8) containing the human serum albumin, ultrafiltering the solution, placing the desired anounts of solution in ampoules, instantly freezing the solution in liquid nitrogen, drying the frozen solution by lyophilization and sealing the ampoules.

The practice of the present invention is further illustrated by the experiments described hereafter.

Crystalline type A botulinum toxin is produced in deep culture. It is a high-molecular-weight protein aggregate of about 900,000. It dissociates under certain conditions of pH and ionic strength into a neurotoxic component of about 150,000 molecular weight and one or more nontoxic protein that have an important role in the stability of the neurotoxin component. High-quality crystalline type A toxin produced from the Hall A strain has a specific toxicity in mice of $\geq 3 \times 10^7$ U/mg, an $A_{260}/A_{278}$ ratio of less than 0.60, and a distinct pattern of banding on gel electrophoresis.

The crystalline botulinum type A toxin for the following studies was prepared by the same procedure used for the manufacture of the toxin in the current commercial product. The toxin preparation was crystallized twice and had an $A_{260}/A_{278}$ ratio of 0.53, indicating that there was very little, if any, contaminating nucleic acid present. The specific toxicity of the toxin dissolved in 50 mM sodium phosphate (pH 6·8) was 28.4 U/ng as calculated by the method of Schantz and Kautter described in Assoc. Official Anal. Chem. 61, p. 96 (1978). The stock supply of toxin suspended in the mother liquor of the second crystallization was adjusted to a concentration of 4 milligrams per ml and stored at 4°C or lower. For drying experiments, a sample of toxin crystals was diluted 10-fold in 50 mM sodium phosphate buffer (pH 6.8) and held at room temperature for -3 h with occasional gentle mixing by inversion to dissolve the crystals. This solution was further diluted 1:100 in 30 mM sodium phosphate-0.2% gelatin (pH 6.2) (gel-phosphate), and the toxin titer was approximated by determining the time to death after intravenous injection.

To evaluate the effect of different stabilizers, the original 1:10 dilution was then diluted to the desired level of toxicity (usually 1,000 U/ml) in a solution containing the stabilizing compound to be tested (see Table 1 for the compounds and concentrations tested). The toxicity of the solutions to be lyophilized were determined by diluting the toxin-excipient combinations in gel-phosphate and injecting 0.5 ml of each dilution intraperitoneally into 18- to 22-g white mice as described above.

For lyophilization, aliquots (usually 0.1 ml) of the toxin solutions were pipetted into 0.5 dram (ca. 1.85-ml), screw-cap glass vials with rubber-lined closures. The solutions in glass vials were then frozen in a bath of liquid nitrogen at -200°C or in some experiments by placing them in a freezer at -20°C or -70°C. The loosely capped vials were then placed into a 150 ml lyophilization flask that had been precooled in liquid nitrogen. The lyophilization flask was partially immersed in liquid nitrogen and connected to a lyophilizer (Virtis Freezemobile 12, Virtis Co., Inc. Gardiner, N.Y.). The liquid nitrogen was maintained in contact with the lyophilization flask until the pressure dropped to approximately 30 millitorrs (ca. 4.0 kPa). The liquid nitrogen jacket was then removed, the flask and contents were allowed to come to room temperature (ca. 3 to 4 h), and the drying procedure was continued for 12 to 18 h. The pressure was maintained at or below 20 millitorrs for the remainder of the cycle. Condensor temperature on the lyophilizer was constant at ca. -60°C. After the drying cycle was completed, the lyophilized toxin vials were removed from the flask, tightly capped, and held at room temperature for up to 3 days until the mouse assay.

The lyophilized preparations were usually reconstituted in 1.0 ml of distilled water. The use of 0.85% saline as a diluent gave equivalent results. The white cake dissolved immediately and was mixed by gentle inversion of the vials. The resulting solution was transparent and contained no particulates. This solution was titrated by the method used for the prelyophilization solution. The percent recovery (calculated as number of mouse intraperitoneal lethal doses per vial after lyophilization divided by number of mouse intraperitoneal lethal doses per vial before lyophilization x 100) represent averages of trials done in at least duplicate. The variation in independent assays was ca. ±20%

It was found that freezing at -20 or -70°C resulted in greater losses of activity (75 to 90% recovery) as compared with freezing at -200°C in liquid nitrogen (>90% recovery). It was also found that no detectable inactivation ($\leq 20\%$) of type A crystalline toxin ($10^4$ U/ml) occurred during repeated freezing and thawing at -20°C in any of several buffers, including 50 mM sodium phosphate (pH 6.2 to 6.8), 50 mM sodium succinate (pH 6.0), and 50 mM sodium citrate (pH 5.5). However, freezing in acetate buffer results in irreversible loss of toxicity.

It was found that the conditions used for lyophilization had a marked effect on the recovery of toxin. The most critical factor was the absence of sodium chloride in the solution for lyophilization. Leaving sodium chloride out of the solution resulted in recovery of >90% of the starting toxicity. Recovery of the toxin activity was also increased by the addition of human serum albumin (HSA) or bovine serum albumin (BSA) as a stabilizing agent (Table 1). Solutions of BSA or HSA at 9.0 mg/ml had an unadjusted pH of 6.4, at which full recovery of activity was obtained. Full recovery of toxin activity was also obtained when the pH was adjusted to 5.0 or 5.5, and a loss of 20 to 30% was found at pH 7.0 to 7.3 (Table 1). In most experiments, 0.1 ml of solution was lyophilized, whereas drying 0.5 ml (with a reduction in serum albumin concentration to 1.8 mg/ml to maintain a postlyophilization concentration of 0.9 mg/ml after reconstitution) gave slightly lower recoveries (60 to 80% of the initial toxicity).

TABLE 1. Effect of excipients on recovery of toxicity after lyophilization

| Excipient(s) | Toxin concn[a] | pH | % Recovery[b] |
|---|---|---|---|
| Sodium phosphate (50 mM) | 50, 100, 1,000 | 5.0,6.0,6.8 | <10 |
| BSA (5.0 mg/ml)-sodium chloride (9.0 mg/ml) | 20 | 6.4 | 10 |
| BSA (5.0 mg/ml)-sodium chloride (9.0 mg/ml)-mannitol (100 mM) | 20 | 6.4 | 20 |
| BSA (5.0 mg/ml)-sodium chloride (9.0 mg/ml)-trehalose (100 mM) | 20 | 6.4 | <10 |
| BSA (5.0 mg/ml)-sodium chloride (9.0 mg/ml)-sucrose (100 mM) | 20 | 6.4 | 10 |
| BSA (5.0 mg/ml)-sodium chloride (9.0 mg/ml)-glucose (100 mM) | 20 | 6.4 | 10 |
| BSA (5.0 mg/ml)-sodium chloride (9.0 mg/ml)-cellibiose (100 mM) | 20 | 6.4 | 10 |
| BSA (9.0 mg/ml) | 100, 1,000 | 6.4 | 88, 75 |
| BSA (9.0 mg/ml)-sodium citrate (50 mM) | 100, 1,000 | 5.0 | >90, >90 |
| BSA (9.0 mg/ml)-sodium phosphate (50 mM) | 100, 1,000 | 5.5 | >90, >90 |
| BSA (9.0 mg/ml)-sodium phosphate (50 mM) | 1,000 | 7.3 | 60 |
| HSA (9.0 mg/ml) | 100, 1,000 | 6.4 | >90, >90 |

[a]Type A mouse intraperitoneal lethal doses per vial before lyophilization.
[b](Number of mouse lethal doses after lyophilization/number mouse lethal doses before lyophilization) x 100.

EP 0 593 176 A2

The results of these studies indicate that the amount of inactive toxin or toxoid formed during preparation can be decreased by elimination of NaCl and use of a lower pH. Pharmaceutical compositions made by the method of the present invention retained their full activity after lyophilization. In contrast, it has been reported that the commercially available pharmaceutical compositions of type A toxin suffer considerable loss of activity (about 90%) during dilution and drying. As previously stated, the reduction in the amount of inactivated toxin is important because it can act as a toxoid and lead to antibody formation and patient immunity.

It will be apparent to those skilled in the art that a number of changes and modifications can be made without departing from the spirit and scope of the invention. For example, although HSA is preferred as a stabilizer, it may be desirable to use BSA or another protein or low-molecular-weight excipient for toxin stabilization, since certain unknown contaminants may be present in human blood proteins.

## Claims

1. A pharmaceutical composition consisting essentially of lyophilized active botulinum toxin type A, said composition being free of sodium chloride.

2. A composition according to claim 1 which contains less than about 25 % inactive toxin.

3. A composition according to claim 1 or 2 which also contains serum albumin.

4. A lyophilized pharmaceutical composition consisting essentially of botulinum toxin type A and a stabilizing amount of serum albumin, said composition being sodium chloride-free with a pH of 6.2 to 6.8 and being substantially free of inactive toxin.

5. A method of preparing a lyophilized botulinum toxin containing pharmaceutical composition which comprises:
   (a) dissolving botulinum toxin in distilled water or a sodium chloride-free buffer solution;
   (b) placing the resulting solution in a container;
   (c) freezing the solution in the container at a temperature below about -70°C; and
   (d) drying the solution and removing the water therefrom by lyophilization to obtain the desired composition.

6. A method according to claim 5 in which, in step (a), the solution contains a protein stabiliser.

7. A method according to claim 6 in which the protein stabiliser is serum albumin.

8. A method according to any one of claims 5 to 7 in which the pH of the buffer solution in step (a) is 5.0 to 6.8.